# EUROPEAN PATENT SPECIFICATION

(11) **EP 3 378 453 B1**
(45) Date of publication and mention of the grant of the patent: **25.12.2019**
(21) Application number: 15908745.1
(22) Date of filing: 18.11.2015
(51) Int. Cl.: A61F 13/49, A61F 13/496

(54) **WEARABLE ARTICLE**
WEARABLE-ARTIKEL
ARTICLE À PORTER

(43) Date of publication of application: 26.09.2018
(73) Proprietor: Unicharm Corporation, Shikokuchuo-shi, Ehime 799-0111 (JP)
(72) Inventor: NAGASE, Noriko, Kanonji-shi Kagawa 769-1602 (JP); NGUYEN, Thi Yen Minh, Kanonji-shi Kagawa 769-1602 (JP); UEDA, Masumi, Kanonji-shi Kagawa 769-1602 (JP)
(74) Representative: Staeger & Sperling Partnerschaftsgesellschaft mbB
(86) International application number: PCT/JP2015/082383
(87) International publication number: WO 2017/085805

(56) References cited:
- EP-A1- 2 415 430
- WO-A1-96/10481
- WO-A1-2015/041928
- JP-A- 2005 124 854
- JP-A- 2010 240 112
- JP-A- 2014 188 189
- JP-A- 2014 221 122
- JP-A- 2015 107 223
- JP-U- 3 166 677

## Description

### [Technical Field]

The present invention relates to a wearable article, namely a diaper.

### [Background Art]

A wearable article which is put on the body (the lower part) of a user (e.g., pull-on disposable diaper) has been conventionally known. Generally speaking, a wearable article such as a disposable diaper includes a stretchable, waist exterior member which covers wearer's waist. When such a wearable article is put on a wearer, the stretchability of the waist exterior member increases fit of wearer's waist, so that the wearable article is not displaced. But, in order to prevent rash of wearer's skin due to tightness of the waist exterior member having such a stretchability, there is known a technique that providing slits (cuts) on a surface of the waist exterior member ensures air permeability. For example, PTL 1 discloses a pull-on wearable article having a pattern formed by providing X-shaped slits or V-shaped slits, making it possible to achieve both of air permeability and visual recognition of the pattern.

### [Citation List]

### [Patent Literature]

[PTL 1] Japanese Patent Application Publication No. 2015-89447 Further prior art in this technical field is disclosed in document WO 2015/041928 A1.

### [Summary of Invention]

### [Technical Problem]

When a pull-on wearable article described in PTL 1 is worn, an exterior member is stretched in the lateral direction and then cuts open, improving the visibility of a pattern formed by the cuts. But, in PTL 1, whereas the cuts are distributed evenly (e.g., in a grid pattern), a force exerted on the cuts when a wearer puts on the wearable article is not necessarily uniform. Consequently, the visibility of the cuts can be insufficiently in such a manner that some cuts are less likely to open, or that openings of the cuts are not uniform.

In view of the problem described above, it is an advantage of the present invention to provide a wearable article including cuts whose visibility is sufficiently good.

### [Solution to Problem]

The present invention to achieve the above advantage is defined in claim 1 and refers to a wearable article being a diaper having a longitudinal direction, a lateral direction, and a thickness direction that intersect one another, the wearable article including: a front exterior member arranged along the lateral direction; a back exterior member arranged along the lateral direction; a plurality of elastic members extending along the lateral direction; and a plurality of line-shaped cuts along the lateral direction having a predetermined lateral length. Each of the front exterior member and the back exterior member includes a non-skin-side sheet located on a non-skin side and a skin-side sheet located on a skin side. The plurality of elastic members are provided lined in the longitudinal direction between the non-skin-side sheet and the skin-side sheet. The plurality of cuts are disposed of at least either one of the non-skin-side sheet and the skin-side sheet of at least either one of the front exterior member and the back exterior member. A first cut of the plurality of cuts is provided between a first elastic member and a second elastic member adjacent in the longitudinal direction of the plurality of elastic members. A distance between the first cut and the first elastic member is smaller than a distance between the first cut and the second elastic member. A second cut of the plurality of cuts is provided between the second elastic member and a third elastic member adjacent in the longitudinal direction of the plurality of elastic members. A distance between the second cut and the third elastic member is smaller than a distance between the second cut and the second elastic member. The first cut and the second cut overlap in the lateral direction, and a no cut region in the longitudinal direction is provided between the first cut and the second cut.

Other features of this invention will become apparent from the description in this specification and the attached drawings.

### [Advantageous Effects of Invention]

According to the present invention, it is possible to provide a wearable article including cuts whose visibility is sufficiently good.

### [Brief Description of Drawings]

FIG. 1 is a schematic perspective view of a disposable diaper 1 (diaper 1), which is an example of an absorbent article according to the present embodiment.
FIG. 2 is a schematic plan view of a diaper 1 with being spread out, as viewed from the skin side.
FIG. 3 is a cross-sectional view of a diaper 1 taken along line A-A in FIG. 2.
FIG. 4 is a diagram illustrating arrangement of slits 50 disposed of the front exterior member 30.
FIG. 5 is a magnified view of the region X of FIG. 4.
FIG. 6 is a diagram schematically showing the openings of a first cut 51 and a second cut 52.

### [Description of Embodiments]

At least the following matters will be made clear by the description in the present specification and the accompanying drawings.

A wearable article having a longitudinal direction, a lateral direction, and a thickness direction that intersect one another, the wearable article including: a front exterior member arranged along the lateral direction; a back exterior member arranged along the lateral direction; a plurality of elastic members extending along the lateral direction; and a plurality of cuts having a predetermined lateral length. Each of the front exterior member and the back exterior member includes a non-skin-side sheet located on a non-skin side and a skin-side sheet located on a skin side. The plurality of elastic members are provided lined in the longitudinal direction between the non-skin-side sheet and the skin-side sheet. The plurality of cuts are disposed of at least either one of the non-skin-side sheet and the skin-side sheet of at least either one of the front exterior member and the back exterior member. A first cut of the plurality of cuts is provided between a first elastic member and a second elastic member adjacent in the longitudinal direction of the plurality of elastic members. A distance between the first cut and the first elastic member is smaller than a distance between the first cut and the second elastic member. A second cut of the plurality of cuts is provided between the second elastic member and a third elastic member adjacent in the longitudinal direction of the plurality of elastic members. A distance between the second cut and the third elastic member is smaller than a distance between the second cut and the second elastic member. The first cut and the second cut overlap in the lateral direction, and no cut is provided between the first cut and the second cut.

With such a wearable article, either one of the upper and lower edges of each cut is located closer to an elastic member, and is more subject to large tension of that elastic member. The other one of the upper and lower edges is located away from the elastic member, and a region having no cut is formed, making it difficult for the other edge to be subject to the tension of the elastic member. This makes it easier for the cut to open vertically when a wearer put on the wearable article, improving the visibility of the cut. That is, it is possible to provide a wearable article including the cut whose visibility is sufficiently good.

In such a wearable article, it is preferable
that the wearable article further comprises a zeroth elastic member and a fourth elastic member, the zeroth elastic member being adjacent to and located longitudinally above the first elastic member, fourth elastic member being adjacent to and located longitudinally below the third elastic member,
that a third cut is provided on a side opposite to the first cut across the first elastic member, at least a part of the third cut overlapping the first cut in the lateral direction,
that a distance between the third cut and the first elastic member is smaller than a distance between the third cut and the zeroth elastic member,
that a fourth cut is provided on a side opposite to the second cut across the third elastic member, at least a part of the fourth cut overlapping the second cut in the lateral direction, and
that a distance between the fourth cut and the third elastic member is smaller than a distance between the fourth cut and the fourth elastic member.

With such a wearable article, two pairs of cuts are symmetrically formed across corresponding elastic member: a pair of the first cut and the third cut; and a pair of the second cut and the fourth cut. These two pairs of cuts together open vertically when a wearer puts on a wearable article. This makes it easier to visually recognize the cuts as two pairs of openings, making it possible to further improve the visibility of each cut (slit 50).

In such a wearable article, it is preferable
that a fifth cut is provided between the second elastic member and the third elastic member,
that a distance between the fifth cut and the second elastic member is smaller than a distance between the fifth cut and the third elastic member,
that a sixth cut is provided between the third elastic member and the fourth elastic member,
that a distance between the sixth cut and the fourth elastic member is smaller than a distance between the sixth cut and the third elastic member,
that the fifth cut and the sixth cut both have no overlap in the lateral direction with all of the first cut, the second cut, the third cut, and the fourth cut,
that the fifth cut and the sixth cut overlap in the lateral direction, and
that the cut is not provided between the fifth cut and the sixth cut.

With such a wearable article, in a similar manner to the first cut and the second cut, the fifth cut and the sixth cut open when a wearer puts on the wearable article. In this case, the fifth cut and the sixth cut are arranged respectively near different elastic members from the first cut and the second cut. Accordingly, the tension of each elastic member is efficiently exerted on corresponding cut, make it easier to open the cut. This makes it possible to further improve the visibility of the cuts.

In such a wearable article, it is preferable
that the wearable article further comprises a fifth elastic member that is adjacent to and located longitudinally below the fourth elastic member,
that a seventh cut is provided at a position which is located on a side opposite to the fifth cut across the second elastic member, and at least a part of which overlaps the fifth cut in the lateral direction,
that a distance between the seventh cut and the second elastic member is smaller than a distance between the seventh cut and the first elastic member,
that a eighth cut is provided at a position which is located on a side opposite to the sixth cut across the fourth elastic member, and at least a part of which overlaps the sixth cut in the lateral direction, and
that a distance between the eighth cut and the fourth elastic member is smaller than a distance between the eighth cut and the fifth elastic member.

With such a wearable article, two pairs of cuts are symmetrically formed across corresponding elastic member: a pair of the fifth cut and the seventh cut; and a pair of the sixth cut and the eighth cut. These two pairs of cuts open together with two pairs of the cuts composed of the first to fourth cuts. This makes it easier to visually recognize four pairs of the cuts, and making it possible to further improve the visibility of each cut.

In such a wearable article, it is preferable that a set of the first cuts, a set of the second cuts, a set of the third cuts, a set of the fourth cuts, a set of the fifth cuts, a set of the sixth cuts, a set of the seventh cuts and a set of the eighth cuts are each arranged periodically in the lateral direction.

With such a wearable article, each set of the first cuts 51 to the eighth cuts 58 is arranged periodically in the lateral direction. Accordingly the cuts each having a good visibility are formed across a lateral, wide area of the front (back) exterior member, making it possible to improving the visibility of cuts throughout the exterior member. Arranging the cuts in substantially a zig-zag pattern improves the design of the entirety of the front (back) exterior member.

In such a wearable article, it is preferable that among the plurality of cuts, concerning a distance between a certain cut and the elastic member arranged nearest the certain cut, and a distance between another cut and the elastic member arranged nearest the other cut, a difference between the distances is equal to or less than 1.0 mm.

With such a wearable article, it is difficult to differ significantly the magnitude of tension exerted on each cut by its corresponding elastic member. Accordingly, the degree of opening of each cut can be made substantially uniform, making it possible to improve both of the visibility and the esthetic appearance of the cuts. In addition, it is possible to suppress such a problem that load concentrates on some of the cuts to tear apart them.

In such a wearable article, it is preferable that the distance between the certain cut and the elastic member arranged nearest the certain cut is equal to the distance between another cut and the elastic member arranged nearest the other cut.

With such a wearable article, the magnitude of tension exerted on each cut by each elastic member can be uniform. Accordingly, the degree of opening of each cut becomes uniform, making it possible to further improve the visibility and the esthetic appearance of the cuts.

In such a wearable article, it is preferable that the wearable article includes a portion in which an interval between two cuts adjacent in the lateral direction is larger than a length of each of the two cuts.

With such a wearable article, it is possible to prevent the adjacent cuts from being torn apart by a force applied when a wearer puts on the wearable article and pulls it up from his/her legs toward his/her crotch, making it easier to maintain the strength of the front (back) exterior member.

In such a wearable article, it is preferable that an interval between two cuts adjacent in the lateral direction is larger than a length of each of the two cuts.

Such a wearable article prevents adjacent cuts from being torn apart together by a force applied when a wearer puts on the wearable article and pulls it up from his/her legs toward his/her crotch. It is possible to ensure, in a region between each pair of laterally adjacent cuts, a large no cut region in which no cut is provided. This makes it easier to open the cuts located in the longitudinal ends of the no cut region.

In such a wearable article, it is preferable that the cut extends through the skin-side sheet in the thickness direction.

With such a wearable article, providing the cut in the skin-side sheet, near wearer's skin makes it possible to quickly transfer body heat and sweat of the wearer to the non-skin side. This prevents heat and moisture from being in contact with wearer's skin for long time, making it difficult for the wearer to feel discomfort.

In such a wearable article, it is preferable that the cut extends through the skin-side sheet and the non-skin-side sheet in the thickness direction.

With such a wearable article, when a wearer puts on a wearable article, body heat (heat) and sweat (moisture) of the wearer quickly discharge through the cut from inside of the wearable article (skin side) to outside (non-skin side). This suppresses problems caused when the wearable article is worn, such as stuffiness, rash of wearer's skin, making it more difficult for the wearer to feel discomfort.

In such a wearable article, the cut is line-shaped along the lateral direction.

With such a wearable article, the cut is line-shaped along the lateral direction. This makes it easier for the cuts to open in the longitudinal direction when the elastic member contracts in the lateral direction, improving visibility of the cut.

### = Embodiment =

### < Configuration of Disposable Diaper >

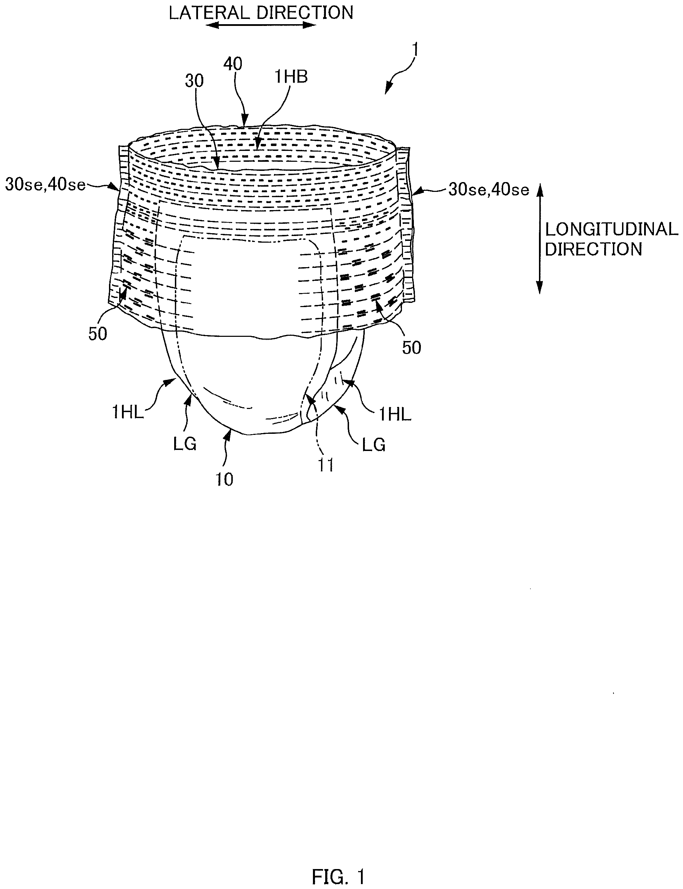

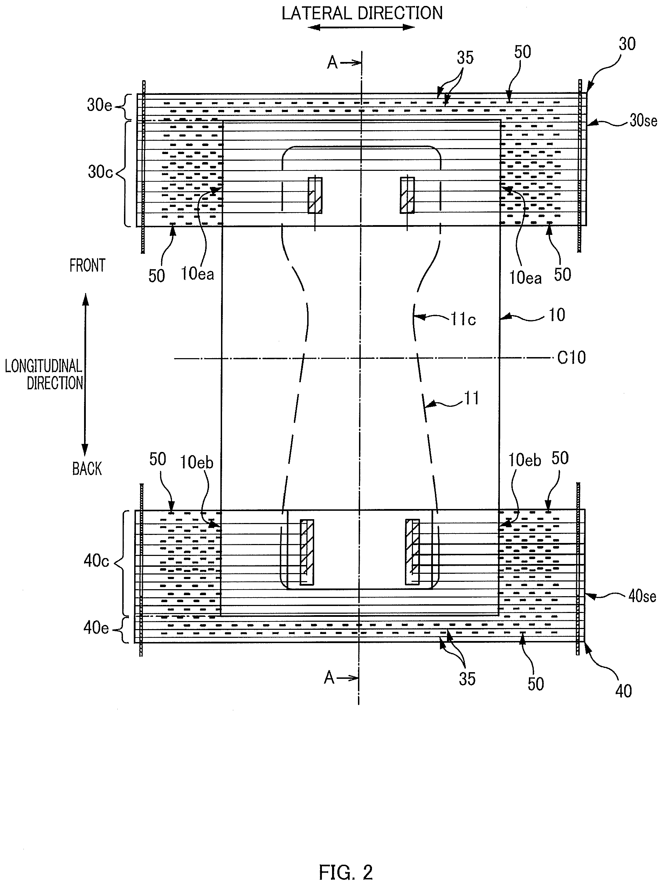

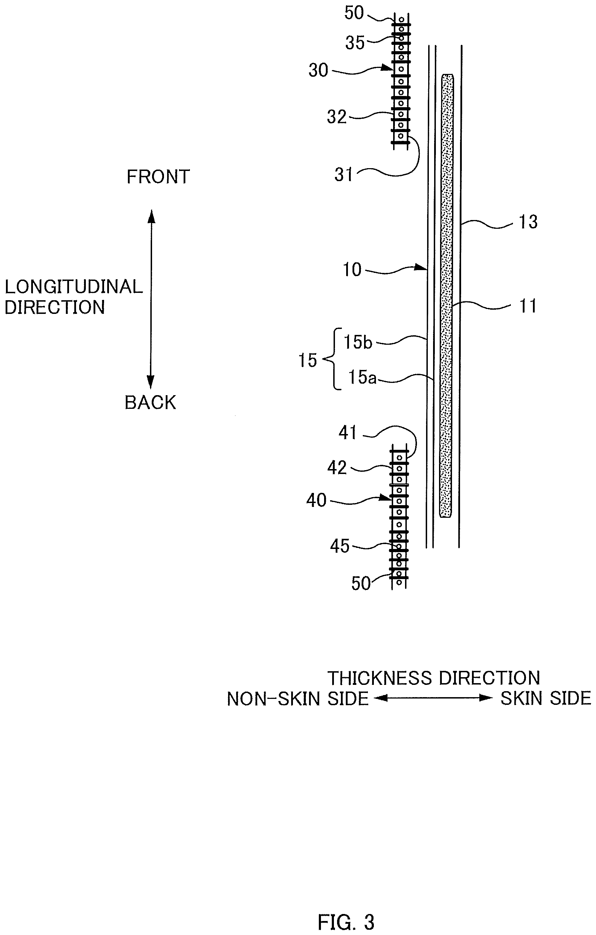

FIG. 1 is a schematic perspective view of a disposable diaper 1 (diaper 1), which is an example of a wearable article according to the present embodiment. FIG. 2 is a schematic plan view of the diaper 1 with being spread out, as viewed from the skin side, FIG. 3 is a cross-sectional view of the diaper 1 taken along line A-A in FIG. 2.

A diaper 1 according to the present embodiment is a so-called 3-piece diaper, and includes three components 10, 30 and 40. That is, the diaper 1 includes: an absorbent main body 10 as a first component, which is brought into contact with a wearer's crotch to absorb excrement such as urine; a front exterior member 30 as a second component, which covers the wearer's front part; and a back exterior member 40 as a third component, which covers the wearer's back part. As shown in FIGS. 2 and 3, the diaper 1 which is spread out has three directions perpendicular to one another: the longitudinal direction; the lateral direction; and the thickness direction. The "front" part in the longitudinal direction is a part which covers the front side of a wearer, and the "back" part is a part which covers the back side of the wearer. In the thickness direction, a side which comes into contact with wearer's skin is defined as a "skin side", and the opposite side is defined as a "non-skin side".

In the diaper 1 which is spread out as shown in FIG. 2, the front exterior member 30 (a upper part in the longitudinal direction in the drawings) and the back exterior member 40 (a lower part in the longitudinal direction in the drawings) are arranged in parallel with being spaced in the longitudinal direction. And, the absorbent main body 10 is placed extending between the exterior members 30 and 40, and lengthwise ends 10ea and 10eb of the absorbent main body 10 are joined and fixed to their nearest exterior members 30 and 40. Thus, the exterior shape of the diaper 1 is H shape as viewed from above. From this state, the absorbent main body 10 is two-folded on its substantially a lengthwise center C10. In this two-folded state, the front exterior member 30 and the back exterior member 40 which face each other are joined and connected on their front-exterior-member side ends 30se and back-exterior-member side ends 40se (that is, lateral ends, parts to be contact with a wearer's waist), forming a ring shape. Thus, as shown in FIG. 1, a diaper 1 becomes in a state where it is worn, and in which a waist opening 1HB and a pair of leg openings 1HL are formed.

As shown in FIGS. 2 and 3, the absorbent main body 10 includes: an absorbent core 11; a top-face sheet member 13 which covers the absorbent core 11 from the skin side of the absorbent core 11; and a back-face sheet member 15 which covers the absorbent core 11 from the non-skin side of the absorbent core 11 and serves as an exterior of the absorbent main body 10.

The absorbent core 11 is a member formed by depositing liquid absorbent materials, and serves as an absorbent body which absorbs excrement such as urine. As an example of liquid absorbent material, there is provided liquid absorbent fiber such as pulp fiber. For example, the absorbent core 11 may include super absorbent polymer as a liquid-absorbent particulate matter, or may include any liquid absorbent material other than liquid absorbent fibers and liquid-absorbent particulate matters. The absorbent core 11 may be covered with a liquid-permeable sheet (not shown) such as tissue paper. The absorbent core 11 of the present embodiment includes a necking portion 11c between its longitudinal front end and its longitudinal back end in FIG. 2, and the lateral width of the necking portion 11c is smaller than the lateral widths of the longitudinal front and back ends. Thus, as shown in FIG. 2, the absorbent core 11 has an hourglass shape as viewed from above.

The top-face sheet member 13 is a sheet-shaped member covering the absorbent core 11 from the skin side; for example a liquid-permeable nonwoven fabric having a planar size larger than the absorbent core 11. The back-face sheet member 15 is a sheet-shaped member covering the absorbent core 11 from the non-skin side and has a planar size larger than the absorbent core 11; it is exemplified by a two-layered sheet formed by attaching an exterior sheet 15b made of nonwoven fabric and a liquid-impermeable leak-proof sheet 15a made of polyethylene, polypropylene, or the like. Between the back-face sheet member 15 and the top-face sheet member 13, the absorbent core 11 is sandwiched. And, the back-face sheet member 15 and the top-face sheet member 13 are attached to each other in a frame-like manner on their respective parts projecting beyond the four edges of the absorbent core 11, thereby forming the absorbent main body 10. The back-face sheet member 15 may be formed of only a leak-proof sheet 15a, not having an exterior sheet 15b.

In a diaper 1, a so-called barrier cuff or a leg gather (both not shown) may be formed of the exterior sheet 15b, etc. The barrier cuff is, for example, a leakage-proof wall standing in each of the lateral ends of the top-face sheet member 13, and the leg gather is a stretchable leg-around portion formed in each of the leg openings 1HL and 1HL of the diaper 1. It is also acceptable that, as a second sheet, a liquid-permeable sheet such as tissue paper is placed between the top-face sheet member 13 and the absorbent core 11, or between the back-face sheet member 15 and the absorbent core 11.

The front exterior member 30 and the back exterior member 40 are both sheet members whose shapes are a rectangle as viewed from above, and are each made of a flexible sheet such as nonwoven fabric. As shown in FIG. 3, the front exterior member 30 is formed by stacking and joining a skin-side sheet 31 and a non-skin-side sheet 32 from the skin side in the thickness direction. Similarly, the back exterior member 40 is formed by stacking and joining a skin-side sheet 41 and a non-skin-side sheet 42 from the skin side in the thickness direction. The front exterior member 30 and the back exterior member 40 are stacked onto the longitudinal ends 10ea and 10eb of the absorbent main body 10 from the non-skin side of the absorbent main body 10, and are joined to the ends 10ea and 10eb.

When placing the absorbent main body 10 onto the front exterior member 30, the absorbent main body 10 is not placed on a longitudinal outer, end portion 30e of the front exterior member 30, but the absorbent main body 10 is placed on a portion 30c located close to the longitudinal center compared to the end portion 30e (see FIG. 2). Similarly, when placing the absorbent main body 10 onto the back exterior member 40, the absorbent main body 10 is not placed on a longitudinal outer, end portion 40e of the back exterior member 40, but the absorbent main body 10 is placed on a portion 40c located close to the longitudinal center compared to the end portion 40e. These portions 30e and 40e in which components are not stacked are portions to form the waist opening1HB of the diaper 1 (see FIG. 1). In the portions 30e and 40e, elastic members 35 and 45 (to be described later) are provided in a continuous manner through substantially the entire lateral lengths of the portions 30e and 40e (see FIG. 2).

In predetermined regions of the front exterior member 30 and the back exterior member 40, a plurality of slits 50 are provided at predetermined longitudinal intervals and predetermined lateral intervals. Each of slits 50 is a line-shaped cut of a predetermined lateral length, and extends in the thickness direction through at least either one of (both in the present embodiment) of the exterior members 30 and 40. When a wearer puts on a diaper 1, body heat (heat) and sweat (moisture) of the wearer discharge through the slits 50 from inside of the diaper 1 (skin side) to outside (non-skin side). This suppresses problems caused when a diaper 1 is worn, such as stuffiness, rash of wearer's skin, making it difficult for the wearer to feel discomfort. It is not essential that the slits 50 extend through the exterior members 30 and 40 in the thickness direction. That is, it is sufficient that the slits 50 are formed at least either of the skin-side sheets 31 and 41 or the non-skin-side sheets 32 and 42. In such a configuration, heat and moisture are more likely to be transferred through the slits 50, making it possible to realize the foregoing heat-discharging function and the like. But, in order to efficiently discharge heat and moisture inside the diaper 1 to outside, it is desirable that the slits 50 are provided in at least the skin-side sheets 31 and 41, near wearer's skin. This is for quickly transferring body heat and sweat of a wearer to the non-skin side. This prevents heat and moisture from being in contact with wearer's skin for long time, making it difficult for the wearer to feel discomfort. It should be noted that, even if the slits 50 are provided in only the skin-side sheets 31 and 41, it is possible to visually recognize the slits 50 from outside of the diaper 1 through the non-skin-side sheets 32 and 42. The arrangement of the slits 50 will be described in detail later.

Between two sheets 31 and 32 associated with the front exterior member 30, a plurality of elastic members 35, 35,... (e.g., elastic strings) extending along the lateral direction are placed. And, the plurality of elastic members 35, 35,... are joined and fixed to the sheets 31 and 32 with stretching in the lateral direction. The plurality of elastic members 35, 35,... are provided lined in the longitudinal direction with being spaced longitudinally. Similarly, between two sheets 41 and 42 associated with the back exterior member 40, a plurality of elastic members 45, 45,... (e.g., elastic strings) extending along the lateral direction are placed. The plurality of elastic members 45, 45,... are joined and fixed to the sheets 41 and 42 with stretching in the lateral direction. The plurality of elastic members 45, 45,... are provided lined in the longitudinal direction with being spaced longitudinally. Consequently, the front exterior member 30 and the back exterior member 40 have lateral stretchability, and this stretchability becomes the stretchability of the waist opening1HB of the diaper 1. The elastic members 35 and 45 have substantially the same basic configuration except detailed specification such as thickness, number, longitudinal arrangement and the like.

Some of the elastic members 35 and 45 are separated in a region around the lateral center in which the absorbent core 11 exists (see FIG. 2), and therefore stretchability is not exerted on the region. This suppresses contraction of the absorbent core 11 in the lateral direction (width direction). Accordingly, production of creases is suppressed in the absorbent core 11, making it easier to keep the skin-side surface of the absorbent core 11 substantially flat. Consequently, it is possible to effectively prevent liquid absorption of the absorbent core 11 from being inhibited, making it possible to suppress leakage of excrement which would be caused by creases.

### < Cuts (Slits 50) in Exterior Members 30 and 40>

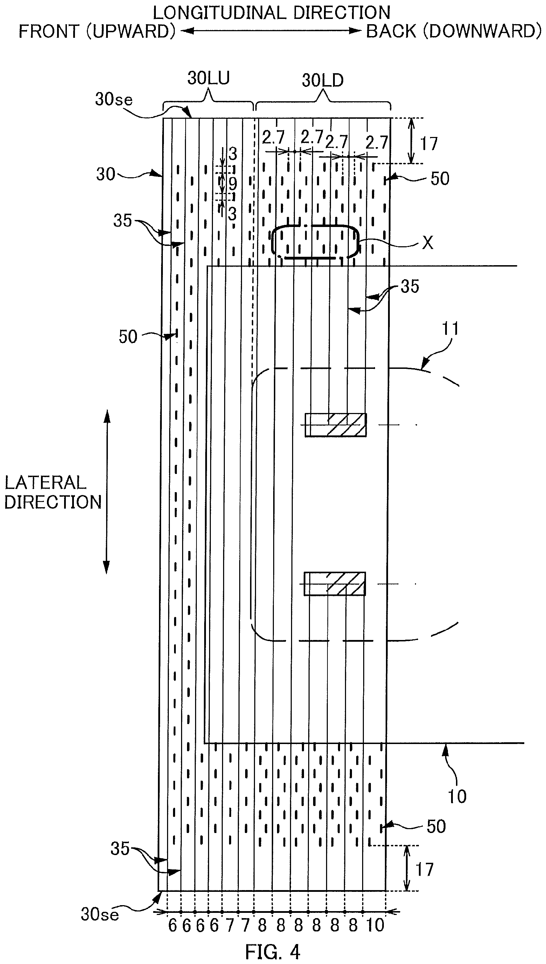

FIG. 4 is a diagram illustrating arrangement of the slits 50 disposed of the front exterior member 30. As shown in FIG. 2, the back exterior member 40 also has the plurality of slits 50, and the configuration and function thereof is substantially the same as those of the front exterior member 30. Accordingly, this section describes mainly the front exterior member 30, the description of the back exterior member 40 is omitted.

As mentioned above, the slits 50 in the present embodiment are "line-shaped" cuts each having a predetermined lateral length. A plurality of the slits 50 are arranged on the front exterior member 30 and between longitudinally adjacent elastic members 35 and 35 with being lined and spaced in the lateral direction. In the example of FIG. 4, the lateral length of each slit 50 (slit length) is 3 mm, and the distance of the space between two slits 50 adjacent in the lateral direction is 9 mm. FIG. 4 is a diagram illustrating an example of arrangement of slits 50 according to the present embodiment, and the number and the sizes (lengths) of the slits 50 are not limited to this example.

A region of the front exterior member 30 in which the front exterior member 30 does not overlap the absorbent core 11 in the longitudinal direction is defined as an upper region 30LU, and a region in which the front exterior member 30 overlaps the absorbent core 11 in the longitudinal direction is defined as a lower region 30LD. The upper region 30LU and the lower region 30LD have different arrangement of the slits 50. Specifically speaking, in the upper region 30LU, between the elastic members 35 and 35 adjacent in the longitudinal direction, the slits 50 are arranged in a single line in the lateral direction. Each slit 50 in the upper region 30LU is arranged in a so-called zig-zag pattern as shown in FIG. 4. On the other hand, in the lower region 30LD, between the elastic members 35 and 35 adjacent in the longitudinal direction, slits 50 are arranged in two lines. That is, in the front exterior member 30, slits 50 in the lower region 30LD are arranged more densely than those in the upper region 30LU.

The term "upper" described herein means the "up" side in the longitudinal direction when a diaper 1 is pants-shaped as shown in FIG. 1. In FIG. 4, the front side in the longitudinal direction corresponds to the "up" side.

The upper region 30LU is a region on which a large force is exerted by being pulled upward with fingers when a wearer puts on the diaper 1. Accordingly, decreasing the number of slits 50 formed in a unit area of the upper region 30LU (density) prevents the strength of the front exterior member 30 from excessively decreasing, suppressing breakage of nonwoven fabric in the region. On the contrary, since the lower region 30LD is less likely to be pulled directly with fingers or the like, increasing the density of slits 50 formed in the region makes it possible to efficiently discharge heat and moisture. That is, the upper region 30LU includes fewer cuts, ensuring strength as a high priority, and the lower region 30LD includes more cuts, ensuring good air permeability.

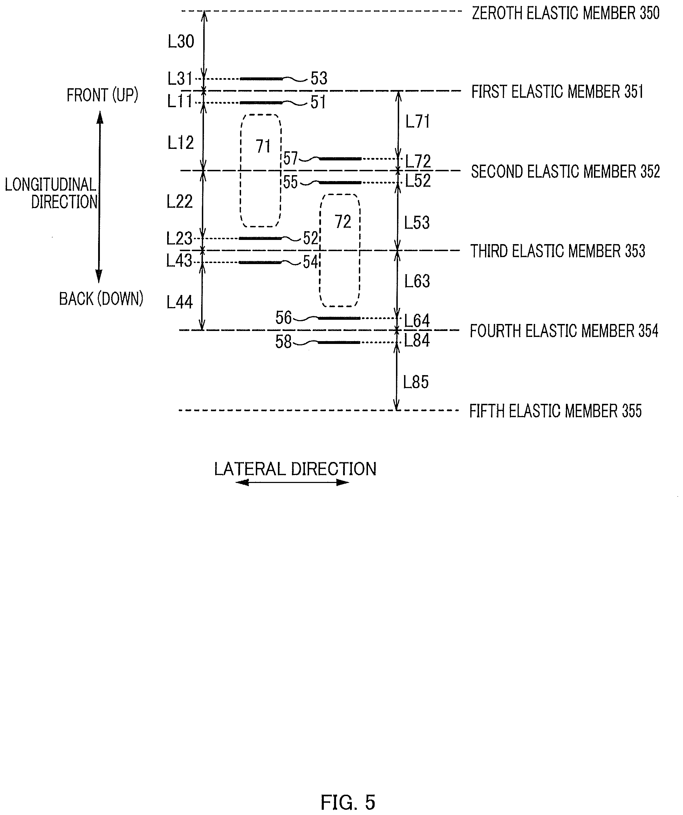

Next, the visibility of the slit 50 is described below. In the present embodiment, adjusting the positions of the slits 50 with respect to each elastic member 35 in the lower region 30LD makes it easier to visually recognize the slits 50 when a wearer puts on the diaper 1. FIG. 5 is a magnified view of the region X of FIG. 4.

In FIG. 5, the four elastic members 35 are arranged lined in an order from up to down in the longitudinal direction: a first elastic member 351, a second elastic member 352, a third elastic member 353, and a fourth elastic member 354. Between the first elastic member 351 and the second elastic member 352 which are adjacent in the longitudinal direction, a first cut 51 (a slit 50) is provided. The first cut 51 is located longitudinally at a position closer to the first elastic member 351 than to the second elastic member 352. In other words, the distance L11 between the first cut 51 and the first elastic member 351 is smaller than the distance L12 between the first cut 51 and the second elastic member 352 (L11 < L12). Similarly, between the second elastic member 352 and the third elastic member 353 which are adjacent in the longitudinal direction, a second cut 52 (a slit 50) is provided. The second cut 52 is located longitudinally at a position closer to the third elastic member 353 than to the second elastic member 352. In other words, the distance L23 between the second cut 52 and the third elastic member 353 is smaller than the distance L22 between the second cut 52 and the second elastic member 352 (L23 < L22) .

Here, the distance between a "cut (slit 50)" and a "elastic member 35" means the distance when the elastic member 35 being stretched in the lateral direction, that is, the longitudinal distance between the elastic member 35 and the slit 50, measured on the front exterior member 30 in a state in which the front exterior member 30 is stretched in the lateral direction against the contraction force of the elastic members 35 and in which the front exterior member 30 is flat and subject to no contraction force of the elastic members 35.

The first cut 51 and the second cut 52 overlap in the lateral direction. In FIG. 5, the first cut 51 and the second cut 52 are located at an identical lateral position. Between the first cut 51 and the second cut 52 in the longitudinal direction, formed is a no cut region 71 in which no slit 50 is provided.

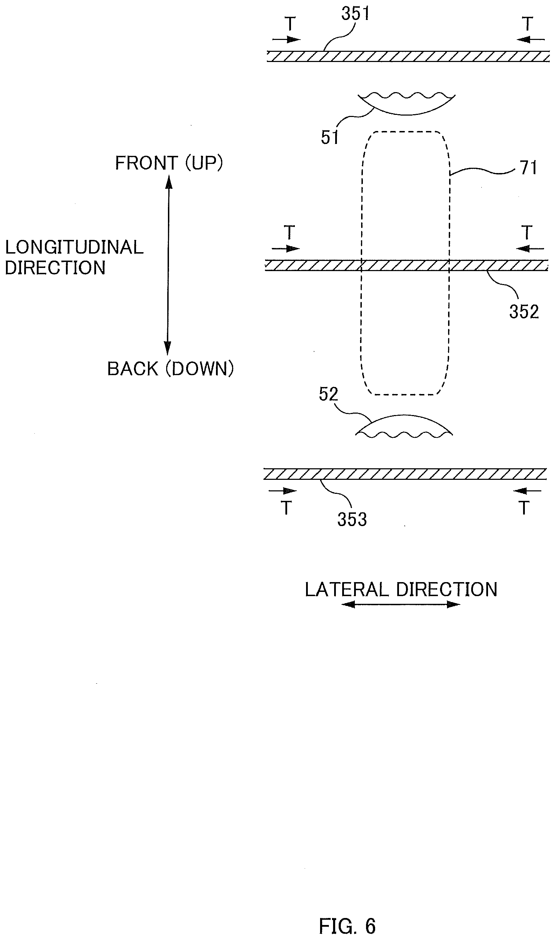

FIG. 6 is a diagram schematically showing the openings of the first cut 51 and the second cut 52. In FIG. 6, the first cut 51 is located closer to the upper, first elastic member 351 than to the lower, second elastic member 352. Accordingly, the first cut is more subject to the tension of the first elastic member 351 (indicated by symbol T in FIG. 6) than to the tension of the second elastic member 352. That is, the upper edge of the first cut 51 is more likely to be subject to the tension of the first elastic member 351, and contraction of the first elastic member 351 makes it easier to form fine creases. In contrast, since the lower edge of the first cut 51 is separated away from the second elastic member 352, the lower edge is less likely to be subject to the tension of the second elastic member 352. Further, in the present embodiment, a no cut region 71 is formed below the first cut 51, and the no cut region 71 has no slit 50 therein, making it easier to maintain the rigidities of the skin-side sheet 31 and the non-skin-side sheet 32 which constitute the front exterior member 30. Accordingly, even if the second elastic member 352 contracts, the contraction does not affect on the first cut 51, making it easier to form moderate creases on the lower edge of the first cut 51 compared to the upper edge of the same. Consequently, as shown in FIG. 6, the first cut 51 forms an opening facing upward, and the opening by the first cut 51 is more likely to be visually recognized when a wearer put on a diaper 1.

Similarly, the second cut 52 is located closer to the lower, third elastic member 353 than to the upper, second elastic member 352. Accordingly, the second cut 52 is more subject to the tension of the third elastic member 353 than to the tension of the second elastic member 352. That is, the lower edge of the second cut 52 is more likely to be subject to the tension of the third elastic member 353, and contraction of the third elastic member 353 makes it easier to form fine creases. In contrast, the upper edge of the second cut 52 is less likely to be subject to the tension of the second elastic member 352. Further, the no cut region 71 is formed above the second cut 52, making it easier to maintain rigidity. Accordingly, contraction of the second elastic member 352 is less likely to affect on the second cut 52, making it easier to form moderate creases on the upper edge of the second cut 52 compared to the lower edge of the same. Consequently, as shown in FIG. 6, the second cut 52 forms an opening facing downward, and the opening by the second cut 52 is more likely to be visually recognized when a wearer puts on the diaper 1.

Back to FIG. 5, on the longitudinal side opposite to the first cut 51 across the first elastic member 351, a third cut 53 is provided which overlaps the first cut 51 in the lateral direction. The third cut 53 is located longitudinally at a position closer to the first elastic member 351 than to zeroth elastic member 350 which is adjacent to and longitudinally above the first elastic member 351. The distance L31 between the third cut 53 and the first elastic member 351 is smaller than the distance L30 between the third cut 53 and zeroth elastic member 350 (L31 < L30).

The third cut 53 is located closer to the lower, first elastic member 351 than to the upper, zeroth elastic member 350. Accordingly, the third cut 53 is more subject to the tension of the first elastic member 351 than to the tension of the zeroth elastic member 350. Further, the no cut region in which no slit 50 is provided is formed above the third cut 53 (not shown in FIG. 6). Accordingly, similar to the second cut 52 shown in FIG. 6, an opening facing downward is more likely to be formed, making it easier to visually recognize the third cut 53 when a wearer puts on the diaper 1. The third cut 53 and the first cut 51 are symmetrically arranged across the first elastic member 351. Therefore, the first cut 51 and the third cut 53 become more likely to be visually recognized as a pair of openings, making it possible to further improve the visibility of each cut (slit 50).

Similarly, on the longitudinal side opposite to the second cut 52 across the third elastic member 353, a fourth cut 54 is provided which overlaps the second cut 52 in the lateral direction. The fourth cut 54 is located longitudinally at a position closer to the third elastic member 353 than to the fourth elastic member 354. The distance L43 between the fourth cut 54 and the third elastic member 353 is smaller than the distance L44 between the fourth cut 54 and the fourth elastic member 354 (L43 < L44).

The fourth cut 54 is located closer to the upper, third elastic member 353 than to the lower, fourth elastic member 354. Accordingly, the fourth cut 54 is more subject to the tension of the third elastic member 353 than to the tension of the fourth elastic member 354. Further, the no cut region in which no slit 50 is provided is formed below the fourth cut 54 (not shown in FIG. 6). Accordingly, similar to the first cut 51 shown in FIG. 6, an opening facing upward is more likely to be formed, making it easier to visually recognize the fourth cut 54 when a wearer puts on the diaper 1. The fourth cut 54 and the second cut 52 are symmetrically arranged across the third elastic member 353, and therefore the second cut 52 and the fourth cut 54 become more likely to be visually recognized as a pair of openings, making it possible to further improve the visibility of each cut.

In FIG. 5, between the second elastic member 352 and the third elastic member 353 which are adjacent in the longitudinal direction, a fifth cut 55 (a slit 50) is provided. The distance L52 between the fifth cut 55 and the second elastic member 352 is smaller than the distance L53 between the fifth cut 55 and the third elastic member 353 (L52 < L53). Similarly, between the third elastic member 353 and the fourth elastic member 354 which are adjacent in the longitudinal direction, a sixth cut 56 (a slit 50) is provided. The distance L64 between the sixth cut 56 and the fourth elastic member 354 is smaller than the distance L63 between the sixth cut 56 and the third elastic member 353 (L64 < L63). The fifth cut 55 overlap the sixth cut 56 in the lateral direction, but does not overlap the first to fourth cuts 51-54 in the lateral direction. In the longitudinal direction, a no cut region 72 in which no slit 50 is provided is formed between the fifth cut 55 and the sixth cut 56.

The fifth cut 55 is arranged close to the second elastic member 352, and the sixth cut 56 is arranged close to the fourth elastic member 354. And, forming a no cut region 72 therebetween realizes a relation between the fifth cut 55 and the sixth cut 56, which is similar to the relation between the first cut 51 and the second cut 52, illustrated in FIG. 6. That is, the fifth cut 55 and the sixth cut 56 open vertically when a wearer puts on a diaper 1, making it easier to visually recognize the fifth cut 55 and the sixth cut 56.

The longitudinal positions of the first cut 51 and the fifth cut 55 do not match, making it easier to open the cuts (slits 50). In the present embodiment, as shown in FIG. 5, the first cut 51 is arranged close to the first elastic member 351, and the fifth cut 55 is arranged close to the second elastic member 352; the longitudinal positions of the first cut 51 and the fifth cut 55 do not match. This makes it easier to exert the tension of the first elastic member 351 on the first cut 51, and to exert the tension of the second elastic member 352 on the fifth cut 55. That is, effectively applying tension to each cut (slit 50) by a corresponding, different elastic member enables to make it easier to open the cut. On the contrary, assuming that the longitudinal positions of the fifth cut 55 and the first cut 51 match, for example, a case where the fifth cut 55 and the first cut 51 are adjacent and located close to the first elastic member 351. In this case, the tension of the first elastic member 351 is exerted on the first cut 51 and the fifth cut 55. The magnitude of tension exerted on the first cut 51 and the fifth cut 55 becomes relatively smaller compared to the foregoing case, making it difficult to open the cuts to fail to improve the visibility sufficiently.

Thus, in the present embodiment, the cuts (slits 50) are arranged in a zig-zag pattern with changing the longitudinal positions thereof; for example, a pair of the first cut 51 and the fifth cut 55 and a pair of the second cut 52 and the sixth cut 56. Accordingly, the tension of the elastic members 35 can be efficiently exerted on the cuts.

On the longitudinal side opposite to the fifth cut 55 across the second elastic member 352, a seventh cut 57 is provided which overlaps the fifth cut 55 in the lateral direction (see FIG. 5). The seventh cut 57 is located longitudinally at a position closer to the second elastic member 352 than to the first elastic member 351. The distance L71 between the seventh cut 57 and the first elastic member 351 is smaller than the distance L72 between the seventh cut 57 and the second elastic member 352 (L71 < L72). On the longitudinal side opposite to the sixth cut 56 across the fourth elastic member 354, a eighth cut 58 is provided which overlaps the sixth cut 56 in the lateral direction (see FIG. 5). The eighth cut 58 is located longitudinally at a position closer to the fourth elastic member 354 than to a fifth elastic member 355 which is adjacent to and longitudinally below the fourth elastic member 354. The distance L84 between the eighth cut 58 and the fourth elastic member 354 is smaller than the distance L85 between the eighth cut 58 and the fifth elastic member 355 (L84 < L85).

The seventh cut 57 and the fifth cut 55 are symmetrically arranged across the second elastic member 352, and the eighth cut 58 and the sixth cut 56 are symmetrically arranged across the fourth elastic member 354. Therefore, the pair of the fifth cut 55 and the seventh cut 57, and the pair of the sixth cut 56 and the eighth cut 58 are each visually recognized as a pair of openings. Aligning these two pairs of openings with two pairs of openings formed of the first to fourth cuts 51-54 makes it easier to visually recognize cuts, and this increases the visibility of each cut.

In the present embodiment, the first cuts 51 to the eighth cuts 58 are provided at a predetermined lateral interval. That is, each set of the first cuts 51 to the eighth cuts 58 is arranged periodically in the lateral direction. Thereby, slits 50 each having a good visibility are formed across a lateral, wide area of the front exterior member 30 (the lower region 30LD) . In the lower region 30LD, the slits 50 are arranged in substantially a zig-zag pattern as shown in FIG. 4, producing design uniformity with the slits 50 arranged in the upper region 30LU, improving esthetic appearance of the entirety of the front exterior member 30.

In the diaper 1 according to the present embodiment, each slit is arranged so that the distance between the slit 50 and its nearest elastic member 35 is substantially uniform. As mentioned above, the diaper 1 includes slits 50 (cuts) between the plurality of elastic members 35 lined in the longitudinal direction; and the magnitudes of respective tensions exerted on the upper and lower edges of each slit 50 is adjusted by adjusting the distances between the slit 50 and an elastic member 35, making it easier to open the slit 50. That is, the degree of opening of each slit 50 is significantly affected by the distance between the slit 50 and an elastic member 35 which applies tension to the slit 50. In diaper 1, the distance between each slit 50 and elastic member 35 which applies tension on the slit 50 (that is, an elastic member 35 arranged nearest the slit 50) is substantially uniform, making it easier to keep the degree of opening of each slit 50 uniform. For example, in FIG. 5, the distance L11 between the first cut 51 and the first elastic member 351 is equal to the following distances: The distance L23 between the second cut 52 and the third elastic member 353; the distance L31 between the third cut 53 and the first elastic member 351; and the distance L43 between the fourth cut 54 and the third elastic member 353. Accordingly, the degree of opening of each cut (slit 50) becomes uniform, making it easier to further improve esthetic appearance. In addition, this makes it easier to suppress such a problem that load concentrates on some of the slit 50 to widen them or to tear apart them.

Desirably, the distance between each slit 50 and its corresponding elastic member 35 is uniform. But, strictly speaking, it is not essential that the distance is uniform, and it is sufficient that each distance is within a predetermined range. That is, among the plurality of cuts, concerning the distance between a certain cut and an elastic member arranged nearest the certain cut (e.g., L11), and the distance between another cut and an elastic member arranged nearest the other cut (e.g., L23), if the difference between these distances are within a predetermined range, these distances can be considered as uniform. In the present embodiment, the distances between each slit 50 and its corresponding elastic member 35 differ by a difference equal to or less than 1.0 mm. Such a degree of the difference makes it difficult to differ significantly the magnitude of tension exerted on each slit 50 by its corresponding elastic member 35, making it possible to keep the degree of opening of each slit 50 substantially uniform.

In the diaper 1 according to in the present embodiment, the space between each pair of longitudinally adjacent elastic members 35 is not uniform as shown in FIG. 4. In this case, the distance between each slit 50 and its nearest elastic member 35 is uniform (2.7 mm in FIG. 4), and this can make the degree of opening of each slit 50 uniform as mentioned above.

In the present embodiment, there are regions in which the distance between two slits 50 adjacent in the lateral direction is larger than the length of each of the two slits 50 (slit length). For example, in FIG. 4, the distance between the slits 50 that are adjacent in the lateral direction is 9 mm, and the length of each slit 50 is 3 mm. Thus, the distance between two cuts (slits 50) is larger than the slit length. This prevents the cuts from being torn apart by a force applied when a wearer puts on the diaper 1 and pulls it up from his/her legs toward his/her crotch, making it easier to maintain the strength of the front exterior member 30.

Further, in the present embodiment, as illustrated in FIG. 5, in a region between two slits 50 adjacent in the lateral direction, a no cut region is formed. The no cut region has a function for improving the visibility of the slits 50 by adjusting contraction around the upper and lower edges of each slit 50 as mentioned above and making it easier to open the slit 50. Accordingly, if the distance between laterally adjacent two slits 50 becomes longer than the slit length, a region for forming a no cut region is more easily ensured. Widening the no cut region makes it possible to further improve the visibility of the slits 50.

No slit 50 is provided in the lateral ends of the front exterior member 30. In FIG. 4, no slit 50 is provided in regions of 17 mm from the respective front-exterior-member side ends 30se, which are the lateral ends of the front exterior member 30. These regions are portions on which a large force is exerted by being pulled toward the lateral sides, for widening the waist opening1HB, by a wearer at the time of putting on the diaper 1. Accordingly, the front exterior member 30 has no slit 50 in the regions of its lateral ends so that the maximum strength of material (nonwoven fabric) is ensured. This makes it possible to suppress breakage of a diaper 1 when a wearer puts on the diaper 1.

### = Other Embodiments =

While the embodiment according to the invention is described above, the above-mentioned embodiment is provided for facilitating the understanding of the present invention, and is not to be interpreted as limiting the present invention. The present invention can be altered and improved without departing from the gist thereof and the present invention includes equivalent thereof. For example, the present invention can be altered as described below.

In the foregoing embodiment, the materials of the exterior sheet 15b, the front exterior member 30, and the back exterior member 40 are nonwoven fabric. However, the present invention is not limited to nonwoven fabric. For example, woven fabric, and any sheet member other than woven fabric may also be employed. And, the exterior sheet 15b may be omitted, and in this case, the leak-proof sheet 15a serves as an exterior of the absorbent main body 10.

In the foregoing embodiment, an elastic string is used as an example of the elastic members 35 and 45. However, the present invention is not limited thereto. For example, band-shaped rubber may be used as the elastic members 35 and 45, and also band-shaped stretchable nonwoven fabric or band-shaped stretchable resin film may be used.

In the foregoing embodiment, the plurality of cuts which are included in the front exterior member 30 and the back exterior member 40 are exemplified by line-shaped slits 50 of a predetermined lateral length (see FIG. 4). However, the shapes of the cuts are not limited thereto. For example, the slit 50 may have a shape of a predetermined longitudinal width. But, if the slit 50 is line-shaped as shown in the foregoing embodiment, this makes it easier to adjust the shape of opening of the slit 50 (see FIG. 6), making it possible to achieve better visibility .

The slit 50 is not required to be arranged extending along the lateral direction, but may be arranged inclined in the lateral direction at a predetermined angle. For example, each slit 50 may be arranged at an inclination angle of about 3°. As long as each slit 50 has the same inclination angle, the condition of the tension exerted on the slit 50 by the elastic members 35 and 45 will be the same. Accordingly, opening the slit 50 enables to improve the visibility.

In the foregoing embodiment, in the front exterior member 30 and the back exterior member 40, slits 50 are evenly arranged in the lateral direction. That is, the distance between slits 50 adjacent in the lateral direction is uniform (9 mm in FIG. 4). However, it is not essential that the length of the space between adjacent slits 50 in the lateral direction is uniform. But, as illustrated in FIG. 6, it is desirable that the lateral positions of a plurality of slits 50 lined in the longitudinal direction (e.g., the first cut 51 to the fourth cut 54 in FIG. 6) match.

### [Reference Signs List]

1 diaper (wearable article, absorbent article), 1HB waist opening, 1HL leg opening,
10 absorbent main body, 10ea end, 10eb end,
11 absorbent core (absorbent body), 11c necking portion,
13 top-face sheet member ,
15 back-face sheet member , 15a leak-proof sheet , 15b exterior sheet,
30 front exterior member,
30e end portion, 30c portion, 30se front-exterior-member side end,
30LU upper region , 30LD lower region,
31 skin-side sheet, 32 non-skin-side sheet,
35 elastic member,
350 zeroth elastic member, 351 first elastic member, 352 second elastic member,
353 third elastic member, 354 fourth elastic member, 355 fifth elastic member,
40 back exterior member,
40e end portion, 40c portion, 40se back-exterior-member side end,
41 skin-side sheet, 42 non-skin-side sheet,
45 elastic member,
50 slit (cut),
51 first cut, 52 second cut, 53 third cut, 54 fourth cut,
55 fifth cut, 56 sixth cut, 57 seventh cut, 58 eighth cut,
71 no cut region, 72 no cut region,
C10 center

## Claims

1. A wearable article being a diaper having a longitudinal direction, a lateral direction, and a thickness direction that intersect one another,
the diaper comprising:
a front exterior member (30) arranged along the lateral direction;
a back exterior member (40) arranged along the lateral direction;
a plurality of elastic members extending along the lateral direction; and
a plurality of line-shaped cuts (50, 51, 52) along the lateral direction having a predetermined lateral length,
each of the front exterior member (30) and the back exterior member (40) including a non-skin-side sheet (32, 42) located on a non-skin side and a skin-side sheet (31, 41) located on a skin side,
the plurality of elastic members being provided lined in the longitudinal direction between the non-skin-side sheet (32, 42 ) and the skin-side sheet (31, 41),
the plurality of cuts (51, 52) disposed of at least either one of the non-skin-side sheet (32) and the skin-side sheet (31) of at least either one of the front exterior member (30) and the back exterior member (40),
a first cut (51) of the plurality of cuts being provided between a first elastic member (351) and a second elastic member (352) adjacent in the longitudinal direction of the plurality of elastic members,
a distance between the first cut (51) and the first elastic member (351) being smaller than a distance between the first cut (51) and the second elastic member (352),
a second cut (52) of the plurality of cuts being provided between the second elastic member (352) and a third elastic member (353) adjacent in the longitudinal direction of the plurality of elastic members,
a distance between the second cut (52) and the third elastic member (353) being smaller than a distance between the second cut (52) and the second elastic member (352),
the first cut (51) and the second cut (52) overlapping in the lateral direction,
a no cut region (71) in the longitudinal direction between the first cut (51) and the second cut (52) in which no cut is provided between the first cut (51) and the second cut (52).

2. A wearable article according to claim 1, wherein
the wearable article further comprises a zeroth elastic member (350) and a fourth elastic member,
the zeroth elastic member (350) being adjacent to and located longitudinally above the first elastic member (351),
the fourth elastic member (354) being adjacent to and located longitudinally below the third elastic member (353),
a third cut (52) is provided on a side opposite to the first cut across the first elastic member (351),
at least a part of the third cut (53) overlapping the first cut in the lateral direction,
a distance between the third cut (53) and the first elastic member (351) is smaller than a distance between the third cut (53) and the zeroth elastic member (350),
a fourth cut (54) is provided on a side opposite to the second cut across the third elastic member,
at least a part of the fourth cut (54) overlapping the second cut (52) in the lateral direction, and
a distance between the fourth cut (54) and the third elastic member (353) is smaller than a distance between the fourth cut (54) and the fourth elastic member (354).

3. A wearable article according to claim 2, wherein
a fifth cut (55) is provided between the second elastic (352) member and the third elastic member (353),
a distance between the fifth cut (55) and the second elastic member (352) is smaller than a distance between the fifth cut (55) and the third elastic member (353),
a sixth cut (56) is provided between the third elastic member and the fourth elastic member (354),
a distance between the sixth cut (56) and the fourth elastic member (354) is smaller than a distance between the sixth cut (56) and the third elastic member (353),
the fifth cut (55) and the sixth cut (56) both have no overlap in the lateral direction with all of the first cut (51), the second cut (52), the third cut (53), and the fourth cut (54),
the fifth cut (55) and the sixth cut (56) overlap in the lateral direction, and
a no cut region (72) in the longitudinal direction between the fifth cut (55) and the sixth cut (56) in which the cut is not provided between the fifth cut (55) and the sixth cut (56).

4. A wearable article according to claim 3, wherein
the wearable article further comprises a fifth elastic member (355) that is adjacent to and located longitudinally below the fourth elastic member (354),
a seventh cut (57) is provided at a position which is located on a side opposite to the fifth cut (55) across the second elastic member (352), and at least a part of which overlaps the fifth cut (55) in the lateral direction,
a distance between the seventh cut (57) and the second elastic member (352) is smaller than a distance between the seventh cut (57) and the first elastic member (351),
a eighth cut (58) is provided at a position which is located on a side opposite to the sixth cut (56) across the fourth elastic member (354), and at least a part of which overlaps the sixth cut (56) in the lateral direction, and
a distance between the eighth cut (58) and the fourth elastic member (354) is smaller than a distance between the eighth cut and the fifth elastic member (355).

5. A wearable article according to claim 4, wherein
a set of the first cuts, a set of the second cuts, a set of the third cuts, a set of the fourth cuts, a set of the fifth cuts, a set of the sixth cuts, a set of the seventh cuts and a set of the eighth cuts are each arranged periodically in the lateral direction.

6. A wearable article according to any one of claims 1 to 5, wherein among the plurality of cuts,
concerning a distance between a certain cut and an elastic member arranged nearest the certain cut, and a distance between another cut and an elastic member arranged nearest the other cut,
a difference between the distances is equal to or less than 1.0 mm.

7. A wearable article according to claim 6, wherein
the distance between the certain cut and the elastic member arranged nearest the certain cut is equal to the distance between another cut and the elastic member arranged nearest the other cut.

8. A wearable article according to any one of claims 1 to 7, wherein
the wearable article includes a portion in which an interval between two cuts adjacent in the lateral direction is larger than a length of each of the two cuts.

9. A wearable article according to claim 8, wherein
the interval between two cuts adjacent in the lateral direction is larger than the length of each of the two cuts.

10. A wearable article according to any one of claims 1 to 9, wherein
the cut extends through the skin-side sheet (31) in the thickness direction.

11. A wearable article according to any one of claims 1 to 10, wherein
the cut extends through the skin-side sheet (31) and the non-skin-side sheet (32) in the thickness direction.

## Patentansprüche

1. Ein Kleidungsstück, das eine Windel mit einer Längsrichtung, einer Querrichtung und einer Dickenrichtung ist, die sich gegenseitig schneiden,
die Windel umfassend:
ein vorderes Außenelement (30), das entlang der Querrichtung angeordnet ist;
ein hinteres Außenelement (40), das entlang der Querrichtung angeordnet ist;
eine Vielzahl von elastischen Elementen, die sich entlang der Längsrichtung erstrecken; und
eine Vielzahl von linienförmigen Schnitten (50, 51, 52) entlang der Querrichtung mit einer vorbestimmten Seitenlänge,
jedes des vorderen äußeren Elements (30) und des hinteren äußeren Elements (40) eine nicht-hautseitige Lage (32, 42), die auf einer nicht-Hautseite angeordnet ist, und eine hautseitige Lage (31, 41), die auf einer Hautseite angeordnet ist, umfasst,
wobei die Vielzahl von elastischen Elementen in Längsrichtung zwischen der nicht-hautseitigen Lage (32, 42) und der hautseitigen Lage (31, 41) ausgefuttert vorgesehen ist,
die Vielzahl von Schnitten (51, 52), die von mindestens einer der nicht-hautseitigen Lage (32) und der hautseitigen Lage (31) von mindestens einem des vorderen äußeren Elements (30) und des hinteren äußeren Elements (40) angeordnet sind,
wobei ein erster Schnitt (51) der Vielzahl von Schnitten zwischen einem ersten elastischen Element (351) und einem zweiten elastischen Element (352) angrenzend in Längsrichtung der Vielzahl von elastischen Elementen vorgesehen ist,
ein Abstand zwischen dem ersten Schnitt (51) und dem ersten elastischen Element (351) kleiner als ein Abstand zwischen dem ersten Schnitt (51) und dem zweiten elastischen Element (352) ist,
wobei ein zweiter Schnitt (52) aus der Vielzahl von Schnitten zwischen dem zweiten elastischen Element (352) und einem dritten elastischen Element (353) angrenzend in Längsrichtung der Vielzahl von elastischen Elementen vorgesehen ist,
ein Abstand zwischen dem zweiten Schnitt (52) und dem dritten elastischen Element (353) kleiner als ein Abstand zwischen dem zweiten Schnitt (52) und dem zweiten elastischen Element (352) ist,
wobei der erste Schnitt (51) und der zweite Schnitt (52) sich in Querrichtung überlappen,
einen Kein-Schnittbereich (71) in Längsrichtung zwischen dem ersten Schnitt (51) und dem zweiten Schnitt (52), in dem kein Schnitt zwischen dem ersten Schnitt (51) und dem zweiten Schnitt (52) vorgesehen ist.

2. Kleidungsstück gemäß Anspruch 1, wobei
das Kleidungsstück ferner ein nulltes elastisches Element (350) und ein viertes elastisches Element umfasst,
wobei das nullte elastische Element (350) angrenzend an und in Längsrichtung über dem ersten elastischen Element (351) angeordnet ist,
wobei das vierte elastische Element (354) angrenzend an und in Längsrichtung unterhalb des dritten elastischen Elements (353) angeordnet ist,
ein dritter Schnitt (52) auf einer dem ersten Schnitt gegenüberliegenden Seite quer durch das erste elastische Element (351) vorgesehen ist,
wobei mindestens ein Teil des dritten Schnittes (53) den ersten Schnitt in Querrichtung überlappt,
ein Abstand zwischen dem dritten Schnitt (53) und dem ersten elastischen Element (351) kleiner ist als ein Abstand zwischen dem dritten Schnitt (53) und dem nullten elastischen Element (350),
ein vierter Schnitt (54) auf einer dem zweiten Schnitt gegenüberliegenden Seite quer durch das dritte elastische Element vorgesehen ist,
wobei mindestens ein Teil des vierten Schnittes (54) den zweiten Schnitt (52) in Querrichtung überlappt, und
ein Abstand zwischen dem vierten Schnitt (54) und dem dritten elastischen Element (353) kleiner ist als ein Abstand zwischen dem vierten Schnitt (54) und dem vierten elastischen Element (354).

3. Kleidungsstück gemäß Anspruch 2, wobei
ein fünfter Schnitt (55) zwischen dem zweiten elastischen (352) Element und dem dritten elastischen Element (353) vorgesehen ist,
ein Abstand zwischen dem fünften Schnitt (55) und dem zweiten elastischen Element (352) kleiner ist als ein Abstand zwischen dem fünften Schnitt (55) und dem dritten elastischen Element (353),
ein sechster Schnitt (56) zwischen dem dritten elastischen Element und dem vierten elastischen Element (354) vorgesehen ist,
ein Abstand zwischen dem sechsten Schnitt (56) und dem vierten elastischen Element (354) kleiner ist als ein Abstand zwischen dem sechsten Schnitt (56) und dem dritten elastischen Element (353),
der fünfte Schnitt (55) und der sechste Schnitt (56) beide keine Überlappung in der Längsrichtung mit allen von dem ersten Schnitt (51), dem zweiten Schnitt (52), dem dritten Schnitt (53) und dem vierten Schnitt (54) aufweisen,
wobei sich der fünfte Schnitt (55) und der sechste Schnitt (56) in der Querrichtung überlappen, und
einen Kein-Schnittbereich (72) in Längsrichtung zwischen dem fünften Schnitt (55) und dem sechsten Schnitt (56), in dem der Schnitt nicht vorgesehen ist, zwischen dem fünften Schnitt (55) und dem sechsten Schnitt (56) aufweisen.

4. Kleidungsstück gemäß Anspruch 3, wobei
das Kleidungsstück ferner ein fünftes elastisches Element (355) umfasst, das angrenzend an und in Längsrichtung unterhalb des vierten elastischen Elements (354) angeordnet ist, ein siebter Schnitt (57) an einer Position vorgesehen ist, die auf einer Seite gegenüber dem fünften Schnitt (55) quer durch das zweite elastische Element (352) angeordnet ist, und von der mindestens ein Teil den fünften Schnitt (55) in der Längsrichtung überlappt,
ein Abstand zwischen dem siebten Schnitt (57) und dem zweiten elastischen Element (352) kleiner ist als ein Abstand zwischen dem siebten Schnitt (57) und dem ersten elastischen Element (351),
ein achter Schnitt (58) an einer Position vorgesehen ist, die auf einer Seite gegenüber dem sechsten Schnitt (56) quer über das vierte elastische Element (354) angeordnet ist und von der mindestens ein Teil den sechsten Schnitt (56) in Querrichtung überlappt, und
ein Abstand zwischen dem achten Schnitt (58) und dem vierten elastischen Element (354) kleiner ist als ein Abstand zwischen dem achten Schnitt und dem fünften elastischen Element (355).

5. Kleidungsstück gemäß Anspruch 4, wobei
ein Satz der ersten Schnitte, ein Satz der zweiten Schnitte, ein Satz der dritten Schnitte, ein Satz der vierten Schnitte, ein Satz der fünften Schnitte, ein Satz der sechsten Schnitte, ein Satz der siebten Schnitte und ein Satz der achten Schnitte jeweils periodisch in der Längsrichtung angeordnet sind.

6. Kleidungsstück gemäß einem der Ansprüche 1 bis 5, wobei
unter der Vielzahl von Schnitten,
bezüglich eines Abstands zwischen einem bestimmten Schnitt und einem elastischen Element, das am nächsten an dem bestimmten Schnitt angeordnet ist, und eines Abstands zwischen einem anderen Schnitt und einem elastischen Element, das am nächsten an dem anderen Schnitt angeordnet ist,
eine Differenz zwischen den Abständen gleich oder kleiner als 1,0 mm ist.

7. Kleidungsstück gemäß Anspruch 6, wobei
der Abstand zwischen dem bestimmten Schnitt und dem elastischen Element, das am nächsten zu dem bestimmten Schnitt angeordnet ist, gleich dem Abstand zwischen einem anderen Schnitt und dem elastischen Element ist, das am nächsten zu dem anderen Schnitt angeordnet ist.

8. Kleidungsstück gemäß einem der Ansprüche 1 bis 7, wobei
der tragbare Gegenstand einen Abschnitt umfasst, in dem ein Abstand zwischen zwei in Querrichtung angrenzenden Schnitten größer ist als eine Länge von jedem der beiden Schnitte.

9. Kleidungsstück gemäß Anspruch 8, wobei
der Abstand zwischen zwei in Querrichtung angrenzenden Schnitten größer ist als die Länge jedes der beiden Schnitte.

10. Kleidungsstück gemäß einem der Ansprüche 1 bis 9, wobei
der Schnitt sich durch die hautseitige Lage (31) in Dickenrichtung erstreckt.

11. Kleidungsstück gemäß einem der Ansprüche 1 bis 10, wobei
der Schnitt sich durch die hautseitige Lage (31) und die nicht hautseitige Lage (32) in Dickheitsrichtung erstreckt.

## Revendications

1. Article à porter qui est une couche ayant une direction longitudinale, une direction latérale et une direction de l'épaisseur qui se croisent les unes les autres, la couche comprenant :
un élément extérieur avant (30) disposé le long de la direction latérale ;
un élément extérieur arrière (40) disposé le long de la direction latérale ;
une pluralité d'éléments élastiques s'étendant le long de la direction latérale; et
une pluralité de découpes en forme de lignes (50, 51, 52) dans la direction latérale ayant une longueur latérale prédéterminée,
chaque élément extérieur avant (30) et chaque élément extérieur arrière (40) comprenant une feuille non côté peau (32, 42) située sur un non côté peau et une feuille côté peau (31, 41) située sur un côté peau,
la pluralité d'éléments élastiques doublés étant prévus dans la direction longitudinale entre la feuille non côté peau (32, 42) et la feuille côté peau (31, 41),
la pluralité de découpes (51, 52) étant disposées sur l'une au moins parmi la feuille non côté peau (32) et la feuille côté peau (31) de l'un au moins parmi l'élément extérieur avant (30) et l'élément extérieur arrière (40),
une première découpe (51) de la pluralité de découpes étant prévues entre un premier élément élastique (351) et un deuxième élément élastique (352) adjacent dans la direction longitudinale de la pluralité d'éléments élastiques,
une distance entre la première découpe (51) et le premier élément élastique (351) étant inférieure à une distance entre la première découpe (51) et le deuxième élément élastique (352),
une deuxième découpe (52) de la pluralité de découpes étant prévues entre le deuxième élément élastique (352) et un troisième élément élastique (353) adjacent dans la direction longitudinale de la pluralité d'éléments élastiques,
une distance entre la deuxième découpe (52) et le troisième élément élastique (353) étant inférieure à une distance entre la deuxième découpe (52) et le deuxième élément élastique (352),
la première découpe (51) et la deuxième découpe (52) se chevauchant dans la direction latérale,
une région sans découpe (71) dans la direction longitudinale entre la première découpe (51) et la deuxième découpe (52) dans laquelle aucune découpe n'est prévue entre la première découpe (51) et la deuxième découpe (52).

2. Article à porter selon la revendication 1, dans lequel
l'article à porter comprend en outre un zéroième élément élastique (350) et un quatrième élément élastique,
le zéroième élément élastique (350) étant adjacent au premier élastique élastique (351) et situé longitudinalement au-dessus de celui-ci,
le quatrième élément élastique (354) étant adjacent au troisième élément élastique (353) et situé longitudinalement en dessous de celui-ci,
une troisième découpe (52) est prévue sur un côté opposé à la première découpe à travers le premier élément élastique (351),
au moins une partie de la troisième découpe (53) chevauchant la première découpe dans la direction latérale,
une distance entre la troisième découpe (53) et le premier élément élastique (351) est inférieure à une distance entre la troisième découpe (53) et le zéroième élément élastique (350),
une quatrième découpe (54) est prévue sur un côté opposé à la deuxième découpe à travers le troisième élément élastique,
au moins une partie de la quatrième découpe (54) chevauchant la deuxième découpe (52) dans la direction latérale, et
une distance entre la quatrième découpe (54) et le troisième élément élastique (353) est inférieure à une distance entre la quatrième découpe (54) et le quatrième élément élastique (354).

3. Article à porter selon la revendication 2, dans lequel
une cinquième découpe (55) est prévue entre le deuxième élément élastique (352) et le troisième élément élastique (353),
une distance entre la cinquième découpe (55) et le deuxième élément élastique (352) est inférieure à une distance entre la cinquième découpe (55) et le troisième élément élastique (353),
une sixième découpe (56) est prévue entre le troisième élément élastique et le quatrième élément élastique (354),
une distance entre la sixième découpe (56) et le quatrième élément élastique (354) est inférieure à une distance entre la sixième découpe (56) et le troisième élément élastique (353),
ni la cinquième découpe (55) et ni la sixième découpe (56) ne présentent aucun chevauchement dans la direction latérale avec toutes les première découpe (51), deuxième découpe (52), troisième découpe (53) et quatrième découpe (54)
la cinquième découpe (55) et la sixième découpe (56) se chevauchent dans la direction latérale, et
une région sans découpe (72) dans la direction longitudinale entre la cinquième découpe (55) et la sixième découpe (56) dans laquelle la découpe n'est pas prévue entre la cinquième découpe (55) et la sixième découpe (56).

4. Article à porter selon la revendication 3, dans lequel
l'article à porter comprend en outre un cinquième élément élastique (355) qui est adjacent au quatrième élément élastique (354) et situé longitudinalement en dessous de celui-ci,
une septième découpe (57) est prévue dans une position située sur un côté opposé à la cinquième découpe (55) à travers le deuxième élément élastique (352), et au moins une partie de celle-ci chevauche la cinquième découpe (55) dans la direction latérale,
une distance entre la septième découpe (57) et le deuxième élément élastique (352) est inférieure à une distance entre la septième découpe (57) et le premier élément élastique (351),
une huitième découpe (58) est prévue dans une position située du côté opposé à la sixième découpe (56) à travers le quatrième élément élastique (354), et au moins une partie de celle-ci chevauche la sixième découpe (56) dans la direction latérale, et
une distance entre la huitième découpe (58) et le quatrième élément élastique (354) est inférieure à une distance entre la huitième découpe et le cinquième élément élastique (355).

5. Article à porter selon la revendication 4, dans lequel
un ensemble des premières découpes, un ensemble des deuxièmes découpes, un ensemble des troisièmes découpes, un ensemble des quatrièmes découpes, un ensemble des cinquième découpes, un ensemble des sixièmes découpes, un ensemble des septièmes découpes et un ensemble des huitièmes découpes sont disposés périodiquement dans la direction latérale.

6. Article à porter selon l'une quelconque des revendications 1 à 5, dans lequel parmi la pluralité de découpes,
concernant une distance entre une certaine découpe et un élément élastique disposé au plus près de la certaine découpe, et une distance entre une autre découpe et un élément élastique disposé au plus près de l'autre découpe,
une différence entre les distances est égale ou inférieure à 1,0 mm.

7. Article à porter selon la revendication 6, dans lequel
la distance entre la certaine découpe et l'élément élastique disposé au plus près de la certaine découpe est égale à la distance entre une autre découpe et l'élément élastique disposé au plus près de l'autre découpe.

8. Article à porter selon l'une quelconque des revendications 1 à 7, dans lequel l'article à porter comprend une partie dans laquelle un intervalle entre deux découpes adjacentes dans la direction latérale est plus grand qu'une longueur de chacune des deux découpes.

9. Article à porter selon la revendication 8, dans lequel
l'intervalle entre deux découpes adjacentes dans la direction latérale est plus grand que la longueur de chacune des deux découpes.

10. Article à porter selon l'une quelconque des revendications 1 à 9, dans lequel la découpe s'étend à travers la feuille côté peau (31) dans la direction de l'épaisseur.

11. Article à porter selon l'une quelconque des revendications 1 à 10, dans lequel la découpe s'étend à travers la feuille côté peau (31) et la feuille non côté peau (32) dans la direction de l'épaisseur.
